# EUROPEAN PATENT SPECIFICATION

(11) **EP 1 608 764 B1**
(45) Date of publication and mention of the grant of the patent: **26.12.2007**
(21) Application number: 04721481.2
(22) Date of filing: 18.03.2004
(51) Int. Cl.: C12P 13/08, C12R 1/19, C12R 1/425, C12N 15/31, C12N 15/52, C12N 15/63

(54) **A PROCESS FOR THE PRODUCTION OF L-AMINO ACIDS USING STRAINS OF THE ENTEROBACTERIACEAE FAMILY WHICH OVEREXPRESS THE GALP GENE, CODING FOR A GALACTOSE PROTON SYMPORTER**
VERFAHREN ZUR HERSTELLUNG VON L-AMINOSÄUREN UNTER VERWENDUNG VON STÄMMEN DER ENTEROBACTERIACEAE FAMILIE WELCHE DAS GALP GEN, DAS FÜR EIN GALAKTOSE-PROTON-SYMPORTER KODIERT, ÜBEREXPRIMIEREN
PROCEDE POUR LA PRODUCTION D'ACIDES AMINES LEVOGYRE UTILISANT DES SOUCHES DE LA FAMILLE DES ENTEROBACTERIACEES SUREXPRIMANT LE GENE GALP, CODANT POUR UN SYMPORTEUR DE PROTONS DE GALACTOSE

(30) Priority: 01.04.2003 DE 10314618
(43) Date of publication of application: 28.12.2005
(73) Proprietor: Evonik Degussa GmbH, 40474 Düsseldorf (DE)
(72) Inventor: RIEPING, Mechthild, 33619 Bielefeld (DE)
(86) International application number: PCT/EP2004/002796
(87) International publication number: WO 2004/087937

(56) References cited:
- EP-A- 0 271 838
- EP-A- 1 149 911
- WO-A-20/04033471
- DE-A- 10 132 946
- US-A- 4 278 765
- VENTER HENRIETTA ET AL: "Molecular dissection of membrane-transport proteins: Mass spectrometry and sequence determination of the galactose-H+ symport protein, GalP, of Escherichia coli and quantitative assay of the incorporation of (ring-2-13C)histidine and 15NH3" BIOCHEMICAL JOURNAL, vol. 363, no. 2, 15 April 2002 (2002-04-15), pages 243-252, XP002288523 ISSN: 0264-6021 cited in the application
- WALMSLEY ADRIAN R ET AL: "8-Anilino-1-naphthalenesulfonate is a fluorescent probe of conformational changes in the D-galactose-H+ symport protein of Escherichia coli" JOURNAL OF BIOLOGICAL CHEMISTRY, vol. 269, no. 25, 1994, pages 17009-17019, XP002288524 ISSN: 0021-9258
- MARTIN GILES E M ET AL: "Forskolin specifically inhibits the bacterial galactose-H+ transport protein, GalP" JOURNAL OF BIOLOGICAL CHEMISTRY, vol. 269, no. 40, 1994, pages 24870-24877, XP002288526 ISSN: 0021-9258
- POSTMA P W: "GALACTOSE TRANSPORT IN SALMONELLA-TYPHIMURIUM" JOURNAL OF BACTERIOLOGY, vol. 129, no. 2, 1977, pages 630-639, XP002288525 ISSN: 0021-9193 cited in the application

### SEQUENCE LISTING

<110> Degussa AG
<120> A process for producing L-amino acids using strains of the Enterobacteriaceae family
<130> 020481 BT
<160> 4
<170> PatentIn version 3.1
<210> 1
   <211> 32
   <212> DNA
   <213> Synthetic sequence
<220>
   <221> Primer
   <222> (1)..(32)
   <223> galP1
<400> 1
   cacaatctag ataaaccata ttggagggca tc 32
<210> 2
   <211> 25
   <212> DNA
   <213> Synthetic sequence
<220>
   <221> Primer
   <222> (1)..(25)
   <223> ga1P2
<400> 2
   gggaggaagc ttggggagat taatc 25
<210> 3
   <211> 1446
   <212> DNA
   <213> Escherichia coli
<220>
   <221> DNA fragment
   <222> (1)..(1446)
   <223> PCR product
<220>
   <221> CDS
   <222> (33)..(1427)
   <223> galP coding region
<400> 3
   cacaatctag ataaaccata ttggagggca tc atg cct gac gct aaa aaa cag      53
                                       Met Pro Asp Ala Lys Lys Gln
                                       1               5
   ggg cgg tca aac aag gca atg acg ttt ttc gtc tgc ttc ctt gcc gct     101
   Gly Arg Ser Asn Lys Ala Met Thr Phe Phe Val Cys Phe Leu Ala Ala
           10                  15                  20
   ctg gcg gga tta ctc ttt ggc ctg gat atc ggt gta att gct ggc gca     149
   Leu Ala Gly Leu Leu Phe Gly Leu Asp Ile Gly Val Ile Ala Gly Ala
       25                  30                  35
   ctg ccg ttt att gca gat gaa ttc cag att act tcg cac acg caa gaa     197
   Leu Pro Phe Ile Ala Asp Glu Phe Gln Ile Thr Ser His Thr Gln Glu
   40                  45                  50                  55
   tgg gtc gta agc tcc atg atg ttc ggt gcg gca gtc ggt gcg gtg ggc     245
   Trp Val Val Ser Ser Met Met Phe Gly Ala Ala Val Gly Ala Val Gly
                   60                  65                  70
   agc ggc tgg ctc tcc ttt aaa ctc ggg cgc aaa aag agc ctg atg atc     293
   Ser Gly Trp Leu Ser Phe Lys Leu Gly Arg Lys Lys Ser Leu Met Ile
               75                  80                  85
   ggc gca att ttg ttt gtt gcc ggt tcg ctg ttc tct gcg gct gcg cca     341
   Gly Ala Ile Leu Phe Val Ala Gly Ser Leu Phe Ser Ala Ala Ala Pro
           90                  95                  100
   aac gtt gaa gta ctg att ctt tcc cgc gtt cta ctg ggg ctg gcg gtg     389
   Asn Val Glu Val Leu Ile Leu Ser Arg Val Leu Leu Gly Leu Ala Val
       105                 110                 115
   ggt gtg gcc tct tat acc gca ccg ctg tac ctc tct gaa att gcg ccg     437
   Gly Val Ala Ser Tyr Thr Ala Pro Leu Tyr Leu Ser Glu Ile Ala Pro
   120                 125                 130                 135
   gaa aaa att cgt ggc agt atg atc tcg atg tat cag ttg atg atc act     485
   Glu Lys Ile Arg Gly Ser Met Ile Ser Met Tyr Gln Leu Met Ile Thr
                   140                 145                 150
   atc ggg atc ctc ggt gct tat ctt tct gat acc gcc ttc agc tac acc     533
   Ile Gly Ile Leu Gly Ala Tyr Leu Ser Asp Thr Ala Phe Ser Tyr Thr
               155                 160                 165
   ggt gca tgg cgc tgg atg ctg ggt gtg att atc atc ccg gca att ttg     581
   Gly Ala Trp Arg Trp Met Leu Gly Val Ile Ile Ile Pro Ala Ile Leu
           170                 175                 180
   ctg ctg att ggt gtc ttc ttc ctg cca gac agc cca cgt tgg ttt gcc     629
   Leu Leu Ile Gly Val Phe Phe Leu Pro Asp Ser Pro Arg Trp Phe Ala
       185                 190                 195
   gcc aaa cgc cgt ttt gtt gat gcc gaa cgc gtg ctg cta cgc ctg cgt     677
   Ala Lys Arg Arg Phe Val Asp Ala Glu Arg Val Leu Leu Arg Leu Arg
   200                 205                 210                 215
   gac acc agc gcg gaa gcg aaa cgc gaa ctg gat gaa atc cgt gaa agt     725
   Asp Thr Ser Ala Glu Ala Lys Arg Glu Leu Asp Glu Ile Arg Glu Ser
                   220                 225                 230
   ttg cag gtt aaa cag agt ggc tgg gcg ctg ttt aaa gag aac agc aac        773
   Leu Gln Val Lys Gln Ser Gly Trp Ala Leu Phe Lys Glu Asn Ser Asn
               235                 240                 245
   ttc cgc cgc gcg gtg ttc ctt ggc gta ctg ttg cag gta atg cag caa        821
   Phe Arg Arg Ala Val Phe Leu Gly Val Leu Leu Gln Val Met Gln Gln
           250                 255                 260
   ttc acc ggg atg aac gtc atc atg tat tac gcg ccg aaa atc ttc gaa        869
   Phe Thr Gly Met Asn Val Ile Met Tyr Tyr Ala Pro Lys Ile Phe Glu
       265                 270                 275
   ctg gcg ggt tat acc aac act acc gag caa atg tgg ggg acc gtg att        917
   Leu Ala Gly Tyr Thr Asn Thr Thr Glu Gln Met Trp Gly Thr Val Ile
   280                 285                 290                 295
   gtc ggc ctg acc aac gta ctt gcc acc ttt atc gca atc ggc ctt gtt        965
   Val Gly Leu Thr Asn Val Leu Ala Thr Phe Ile Ala Ile Gly Leu Val
                   300                 305                 310
   gac cgc tgg gga cgt aaa cca acg cta acg ctg ggc ttc ctg gtg atg       1013
   Asp Arg Trp Gly Arg Lys Pro Thr Leu Thr Leu Gly Phe Leu Val Met
               315                 320                 325
   gct gct ggc atg ggc gta ctc ggt aca atg atg cat atc ggt att cac       1061
   Ala Ala Gly Met Gly Val Leu Gly Thr Met Met His Ile Gly Ile His
           330                 335                 340
   tct ccg tcg gcg cag tat ttc gcc atc gcc atg ctg ctg atg ttt att       1109
   Ser Pro Ser Ala Gln Tyr Phe Ala Ile Ala Met Leu Leu Met Phe Ile
       345                 350                 355
   gtc ggt ttt gcc atg agt gcc ggt ccg ctg att tgg gta ctg tgc tcc       1157
   Val Gly Phe Ala Met Ser Ala Gly Pro Leu Ile Trp Val Leu Cys Ser
   360                 365                 370                 375
   gaa att cag ccg ctg aaa ggc cgc gat ttt ggc atc acc tgc tcc act       1205
   Glu Ile Gln Pro Leu Lys Gly Arg Asp Phe Gly Ile Thr Cys Ser Thr
                   380                 385                 390
   gcc acc aac tgg att gcc aac atg atc gtt ggc gca acg ttc ctg acc       1253
   Ala Thr Asn Trp Ile Ala Asn Met Ile Val Gly Ala Thr Phe Leu Thr
               395                 400                 405
   atg ctc aac acg ctg ggt aac gcc aac acc ttc tgg gtg tat gcg gct       1301
   Met Leu Asn Thr Leu Gly Asn Ala Asn Thr Phe Trp Val Tyr Ala Ala
           410                 415                 420
   ctg aac gta ctg ttt atc ctg ctg aca ttg tgg ctg gta ccg gaa acc       1349
   Leu Asn Val Leu Phe Ile Leu Leu Thr Leu Trp Leu Val Pro Glu Thr
       425                 430                 435
   aaa cac gtt tcg ctg gaa cat att gaa cgt aat ctg atg aaa ggt cgt       1397
   Lys His Val Ser Leu Glu His Ile Glu Arg Asn Leu Met Lys Gly Arg
   440                 445                 450                 455
   aaa ctg cgc gaa ata ggc gct cac gat taa tctccccaag cttcctccc          1446
   Lys Leu Arg Glu Ile Gly Ala His Asp
                   460
<210> 4
   <211> 464
   <212> PRT
   <213> Escherichia coli
<400> 4
   Met Pro Asp Ala Lys Lys Gln Gly Arg Ser Asn Lys Ala Met Thr Phe
   1               5                   10                  15
   Phe Val Cys Phe Leu Ala Ala Leu Ala Gly Leu Leu Phe Gly Leu Asp
               20                  25                  30
   Ile Gly Val Ile Ala Gly Ala Leu Pro Phe Ile Ala Asp Glu Phe Gln
           35                  40                  45
   Ile Thr Ser His Thr Gln Glu Trp Val Val Ser Ser Met Met Phe Gly
       50                  55                  60
   Ala Ala Val Gly Ala Val Gly Ser Gly Trp Leu Ser Phe Lys Leu Gly
   65                  70                  75                  80
   Arg Lys Lys Ser Leu Met Ile Gly Ala Ile Leu Phe Val Ala Gly Ser
                   85                  90                  95
   Leu Phe Ser Ala Ala Ala Pro Asn Val Glu Val Leu Ile Leu Ser Arg
               100                 105                 110
   Val Leu Leu Gly Leu Ala Val Gly Val Ala Ser Tyr Thr Ala Pro Leu
           115                 120                 125
   Tyr Leu Ser Glu Ile Ala Pro Glu Lys Ile Arg Gly Ser Met Ile Ser
       130                 135                 140
   Met Tyr Gln Leu Met Ile Thr Ile Gly Ile Leu Gly Ala Tyr Leu Ser
   145                 150                 155                 160
   Asp Thr Ala Phe Ser Tyr Thr Gly Ala Trp Arg Trp Met Leu Gly Val
                   165                 170                 175
   Ile Ile Ile Pro Ala Ile Leu Leu Leu Ile Gly Val Phe Phe Leu Pro
               180                 185                 190
   Asp Ser Pro Arg Trp Phe Ala Ala Lys Arg Arg Phe Val Asp Ala Glu
           195                 200                 205
   Arg Val Leu Leu Arg Leu Arg Asp Thr Ser Ala Glu Ala Lys Arg Glu
       210                 215                 220
   Leu Asp Glu Ile Arg Glu Ser Leu Gln Val Lys Gln Ser Gly Trp Ala
   225 230 235 240
   Leu Phe Lys Glu Asn Ser Asn Phe Arg Arg Ala Val Phe Leu Gly Val
   245 250 255
   Leu Leu Gln Val Met Gln Gln Phe Thr Gly Met Asn Val Ile Met Tyr
   260 265 270
   Tyr Ala Pro Lys Ile Phe Glu Leu Ala Gly Tyr Thr Asn Thr Thr Glu
   275 280 285
   Gln Met Trp Gly Thr Val Ile Val Gly Leu Thr Asn Val Leu Ala Thr
   290 295 300
   Phe Ile Ala Ile Gly Leu Val Asp Arg Trp Gly Arg Lys Pro Thr Leu
   305 310 315 320
   Thr Leu Gly Phe Leu Val Met Ala Ala Gly Met Gly Val Leu Gly Thr
   325 330 335
   Met Met His Ile Gly Ile His Ser Pro Ser Ala Gln Tyr Phe Ala Ile
   340 345 350
   Ala Met Leu Leu Met Phe Ile Val Gly Phe Ala Met Ser Ala Gly Pro
   355 360 365
   Leu Ile Trp Val Leu Cys Ser Glu Ile Gln Pro Leu Lys Gly Arg Asp
   370 375 380
   Phe Gly Ile Thr Cys Ser Thr Ala Thr Asn Trp Ile Ala Asn Met Ile
   385 390 395 400
   Val Gly Ala Thr Phe Leu Thr Met Leu Asn Thr Leu Gly Asn Ala Asn
   405 410 415
   Thr Phe Trp Val Tyr Ala Ala Leu Asn Val Leu Phe Ile Leu Leu Thr
   420 425 430
   Leu Trp Leu Val Pro Glu Thr Lys His Val Ser Leu Glu His Ile Glu
   435 440 445
   Arg Asn Leu Met Lys Gly Arg Lys Leu Arg Glu Ile Gly Ala His Asp
   450 455 460

## Description

### Field of the Invention

This invention provides a process for the production of L-valine, L-homoserine. L-lysine and L-threonine, using strains of the Enterobacteriaceae family in which the galP gene is overexpressed.

### Background of the Invention

L-amino acids, in particular L-threonine, are used in human medicine and in the pharmaceutical industry, in the foodstuffs industry and very particularly in animal nutrition.

It is known that L-amino acids can be prepared by the fermentation of strains of Enterobacteriaceae, in particular Escherichia coli (E. coli) and Serratia marcescens. As a result of the great importance of this, efforts are constantly made to improve the method of production. Process improvements may relate to fermentation engineering measures such as e.g. stirring and supplying with oxygen, or to the composition of the nutrient media such as e.g. the sugar concentration during fermentation, or to working up to the product form by e.g. ion-exchange chromatography, or the intrinsic performance characteristics of the microorganism itself.

The methods of mutagenesis, selection and mutant choice are used to improve the performance characteristics of these microorganisms. In this way, strains are obtained which are resistant to antimetabolites such as e.g. the threonine analogon α-amino-β-hydroxyvaleric acid (AHV) or auxotrophic for regulatorily important metabolites and which produce L-amino acids such as e.g. L-threonine.

For some time now, the methods of recombinant DNA engineering have also been used for the strain-improvement of L-amino acid-producing strains of the Enterobacteriaceae family, by amplifying individual amino acid biosynthesis genes and testing the effect of this on production. A summary of the information relating to the cellular biology and molecular biology of Escherichia coli and Salmonella can be found in Neidhardt (ed.): Escherichia coli and Salmonella, Cellular and Molecular Biology, 2nd edition, ASM Press, Washington, D.C., USA,(1996).

Venter, Henrietta et al. (Biochemical Journal, vol. 363, no. 2, 15 April 2002 (2002-04-15), pages 243-252, XP002288523 ISSN: 0264-6021) disclose the overexpression of the galP gene (D-galactose-H⁺ symport protein) in E. coli. Walmsley Adrian R et al. (Journal of Biological Chemistry, vol. 269, no. 25, 1994, pages 17009-17019, XP002288524 ISSN: 0021-9258) also disclose the overexpression of the galP gene coding for the D-galactose-H⁺ symport protein in E. coli.

In EP-A-1 149 911 the preparation of L-threonine by fermentation is disclosed, whereby microorganisms of the Enterobacteriaceae family, in particular in E. coli are used which express sucrose PTS or non-PTS genes for sucrose transport. In particular, the lacY type permease, proton transport system, is one of said genes. The subject-matter of the claims differs in that in the present specification the gene which is overexpressed in a microorganism of the Enterobacteriaceae family is the galP gene.

Martin Giles et al. (Journal of Biological Chemistry, vol. 269, no. 40, 1994, pages 24870-24877, XP002288526 ISSN: 0021-9258) showed that forskolin is a remarkably specific inhibitor of energized D-galaktose transport by the GalP sugar-H⁺ symport protein of Escherichia coli. In WO 2004/033471 (published later) a method is disclosed for restoring a Glu⁺ phenotype to a PTS⁻/Glu⁻ bacterial cell which was originally capable of utilizing a phosphotransferase transport system (PTS)for carbohydrate transport.

### Object of the Invention

The inventors have made the object the provision of new measures for the improved fermentative production of L-amino acids, in particular L-threonine

### Summary of the Invention

The invention provides a process for the fermentative production of L-valine, L-valine,L-homoserine, L-lysine and L-threonine, using microorganisms from the Enterobacteriaceae family, in particular those which already produce L-amino acids and in which the nucleotide sequence coding for the galP gene is overexprpssed.

### Detailed Description of the Invention

The gene product coded by the galP gene is known in the prior art, inter alia, as "galactose proton symporter" or "galactose permease".

In general, the protein coded by a nucleotide sequence, i.e. a gene or an allele, or the coded ribonucleic acid is called a gene product.

Alleles are generally understood to be alternative forms of a given gene. The forms are characterized by differences in the nucleotide sequence.

Wherever L-amino acids or amino acids are mentioned in the following, this is intended to mean one or more amino acids, including their salts, chosen from the group consisting of L-threonine, valine and homoserine L-lysine, and L-threonine is particularly preferred.

The word "overexpression", in this connection, describes the increase in intracellular activity or concentration of one or more enzymes or proteins, in a microorganism, which are coded by the corresponding DNA by, for example, increasing the copy number of the gene or genes by at least one (1) copy or using a strong promoter and optionally combining these measures.

As a result of the measure overexpression, the activity or concentration of the corresponding protein is generally increased by at least 10%, 25%, 50%, 75%, 100%, 150%, 200%, 300%, 400% or 500%, at most up to 1000% or 2000%, with respect to the wild type protein or the activity or concentration of the protein in the starting microorganism. The starting microorganism or parent strain is understood to be the microorganism on which the inventive measures are performed.

The invention provides a process for the production of L-amino acids by the fermentation of recombinant microorganisms from the Enterobacteriaceae family, characterized in that
a) the microorganisms producing the above mentioned L-amino acids, in which the galP gene or nucleotide sequences coding for that is overexpressed, is cultivated in a medium under conditions in which said L-amino acid is enriched in the medium or in the cells, and
b) said L-amino acid is isolated, wherein ≥0 to 100 % of the biomass remain in the isolated product

The, in particular recombinant, microorganisms which are also provided by the present invention, can produce L-amino acids from glucose, saccharose, lactose, fructose, maltose, molasses, optionally starch, optionally cellulose or from glycerine and ethanol. They are representatives of the Enterobacteriaceae family chosen from the genera Escherichia, Erwinia, Providencia and Serratia. The genera Escherichia and Serratia are preferred. In the case of the genus Escherichia, the species Escherichia coli is mentioned in particular and in the case of the genus Serratia, the species Serratia marcescens is mentioned in particular.

Recombinant microorganisms are generally produced by transformation, transduction or conjugation using a vector containing the desired gene.

Suitable strains of the genus Escherichia, in particular those producing L-threonine, in particular of the species Escherichia coli are, for example
- Escherichia coli H4581 (EP-A 0 301 572)
- Escherichia coli KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli: BKIIM B-3996 (US-A-5,175,107)
- Escherichia coli kat 13. (WO 98/04715)
- Escherichia coli KCCM-10132 (WO 00/09660)

Suitable strains of the genus Serratia, in particular those producing L-threonine, in particular of the species Serratia marcescens are, for example
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (Applied Biochemistry and Biotechnology 37(3): 255-265 (1992))

L-threonine-producing strains from the Enterobacteriaceae family preferably possess, inter alia, one or more of the genetic or phenotypical features chosen from the group: resistance to a-amino-β-hydroxyvaleric acid, resistance to thialysine, resistance to ethionine, resistance to α-methylserine, resistance to diaminosuccinic acid, resistance to α-aminobutyric acid, resistance to borrelidine, resistance to cyclopentanecarboxylic acid, resistance to rifampicin, resistance to valine analogues such as, for example, valinehydroxamate, resistance to purine analogues such as, for example, 6-dimethyl-aminopurine, a requirement for L-methionine, optionally a partial and compensable requirement for L-isoleucine, a requirement for meso-diaminopimelic acid, auxotrophy with respect to threonine-containing dipeptides, resistance to L-threonine, resistance to threonine raffinate, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity towards fluoropyruvate, defective threonine dehydrogenase, cysteine, resistance to L-valine, sensitivity towards fluoropyruvate, defective threonine dehydrogenase, optionally the ability to utilize saccharose, overexpression of the threonine operon, overexpression of homoserine dehydrogenase I-aspartate kinase I, preferably the feedback resistant form, overexpression of homoserine kinase, overexpression of threonine synthase, overexpression of aspartate kinase, optionally the feedback resistant form, overexpression of aspartate semialdehyde dehydrogenase, overexpression of phosphoenolpyruvate carboxylase, optionally the feedback resistant form, overexpression of phosphoenolpyruvate synthase, overexpression of transhydrogenase, overexpression of the RhtB gene product, overexpression of the RhtC gene product, overexpression of the YfiK gene product, overexpression of a pyruvate carboxylase, and attenuation of acetic acid formation.

It was found that microorganisms of the Enterobacteriaceae family produce L-amino acids in particular chosen from the group L-valine, L-homoserine, E-lysine and L-threonine in an improved manner after overexpression of the galP gene.

The nucleotide sequences of the genes from Escherichia coli are part of the prior art and can be obtained from the genome sequence for Escherichia coli published by Blattner et al. (Science 277: 1453-1462 (1997)).

The galP gene is described, inter alia, by the following data:
- Name:: sugar transporter, galactose-proton symporter
- Function:: as an integral membrane protein, symport of 2-deoxy-D-galactose and a proton into cells
- Reference:: Macpherson et,al.; The Journal of Biological Chemistry 258(7): 4390-4396 (1983), Venter et al.; The Biochemical Journal 363 (Pt 2) 243-252 (2002)

Galactose permease in Salmonella typhimurium is described, inter alia, in the following references:
Postma PW; Journal of Bacteriology 129(2): 630-639 (1977);
Nagelkerke and Postma; Journal of Bacteriology 133(2): 607-613 (1978).

The nucleic acid sequences can be obtained from the databank at the National Center for Biotechnology Information (NCBI) at the National Library of Medicine (Bethesda, MD, USA), the nucleotide sequence databank at the European Molecular Biology Laboratory (EMBL, Heidelberg, Germany and Cambridge,' UK) or from the DNA databank of Japan (DDBJ, Mishima, Japan).

For the sake of better clarity, the nucleotide sequence of the galP gene from Escherichia coli is given as SEQ ID NO:3 and the amino acid sequence of the galactose-proton symporter protein coded by this gene is given as SEQ ID N0:4.

The genes described in the cited literature references can be used in accordance with the invention. Furthermore, alleles of the genes can be used where these are produced by degeneracy of the genetic code or by functionally neutral sense mutations. The use of endogenous genes is preferred.

"Endogenous genes" or "endogenous nucleotide sequences" are understood to be the genes or alleles or nucleotide sequences present in the population of a species.

Included among the alleles which contain functionally neutral sense mutations are those which lead to at least a conservative amino acid exchange in the protein coded by them.

In the case of aromatic amino acids, conservative exchanges are referred to when phenylalanine, tryptophan and tyrosine replace each other. In the case of hydrophobic amino acids, conservative exchanges are referred to when leucine, isoleucine and valine replace each other. In the case of polar amino acids, conservative exchanges are referred to when glutamine and asparagine replace each other. In the case of basic amino acids, conservative exchanges are referred to when arginine, lysine and histidine replace each other. In the case of acidic amino acids, conservative exchanges are referred to when aspartic acid and glutamic acid replace each other. In the case of hydroxyl group-containing amino acids, conservative exchanges are referred to when serine and threonine replace each other.

In the same way, those nucleotide sequences can be used which code for variants of the proteins mentioned which in addition are lengthened or shortened at the N- or C-terminal by at least one (1) amino acid. This extension or reduction amounts to not more than 50, 40, 30, 20, 10, 5, 3 or 2 amino acids or amino acid groupings.

In order to produce an overexpression, the copy number of the corresponding genes can be increased, for example, or the promoter and regulation region or the ribosome binding site which is located upstream of the structural gene can be mutated. Expression cassettes which are incorporated upstream of the structural gene act in the same way. Included among the promoters which can be used are, inter alia, promoters of the lactose and tryptophan operons of Escherichia coli which are known under the names lac and trp promoters. A hybrid promoter which is contained within the -10 region of the lac promoter and the -35 region of the trp promoter is the tac promoter (DeBoer et al.; Proceedings of the National Academy of Sciences of the United States of America 80: 21-25 (1983)). Other promoters which can be used are the left-directed P*_{L}* promoter of the lambda bacteriophages, and promoters of the T7 phages, which interact with a repressor, as also do the lac, trp and tac promoters already mentioned, wherein the transcription cloned genes can be specifically turned on or off. By using inducible promoters, it is also possible to increase expression during the course of fermentative L-threonine production. Expression is also improved by measures to prolong the lifetime of the m-RNA. Furthermore, enzyme activity is also increased by preventing degradation of the enzyme protein. The genes or gene constructs may either be present in plasmids with different copy numbers or be integrated and amplified in the chromosome. Alternatively, furthermore, overexpression of the relevant genes may be achieved by modifying the composition of the medium and culture management.

A person skilled in the art can find instructions for this, inter alia, in Chang and Cohen (Journal of Bacteriology 134: 1141-1156 (1978)), in Hartley and Gregori (Gene 13: 347-353 (1981)), in Amann and Brosius (Gene 40: 183-190 (1985)), in de Broer et al. (Proceedings of the National Academy of Sciences of the United States of America 80: 21-25 (1983)), in LaVallie et al. (BIO/TECHNOLOGY 11: 187-193 (1993)), in WO98/04715, in Llosa et al. (Plasmid 26: 222-224 (1991)), in Quandt and Klipp (Gene 80: 161-169 (1989)), in Hamilton et al. (Journal of Bacteriology 171: 4617-4622 (1989)), in Jensen and Hammer (Biotechnology and Bioengineering 58: 191-195 (1998)) and in well-known textbooks on genetics and molecular biology.

Plasmid vectors which can be replicated in Enterobacteriaceae, such as e.g. cloning vectors derived from pACYC184 (Bartolomé et al.; Gene 102: 75-78 (1991)), pTrc99A (Amann et al.; Gene 69: 301-315 (1988)) or pSC101-derivates (Vocke and Bastia; Proceedings of the National Academy of Sciences USA 80(21): 6557-6561 (1983)) can be used. In a process according to the invention, a strain transformed with a plasmid vector may be used, wherein the plasmid vector contains at least one nucleotide sequence coding for the galP gene

The expression transformation is understood to be the acceptance of an isolated nucleic acid by a host (microorganism).

It is also possible to convert mutations which affect expression of the particular gene by sequence exchange (Hamilton et al.; Journal of Bacteriology 171: 4617-4622 (1989)), conjugation or transduction in different strains.

More detailed explanations of the concepts used in genetics and molecular biology can be found in well-known textbooks of genetics and molecular biology, such as for example the textbook by Birge (Bacterial and Bacteriophage Genetics, 4th ed., Springer Verlag, New York (USA), 2000) or the text book by Berg, Tymoczko and Stryer (Biochemistry, 5th ed., Freeman and Company, New York (USA), 2002) or the manual by Sambrook et al. (Molecular Cloning, A Laboratory Manual, (3-Volume Set), Cold Spring Harbor Laboratory Press, Cold Spring Harbor (USA), 2001).

Furthermore, it may be advantageous for the production of L-amino acids, chosen from the group L-valine, L-homoserine, L-lysine and in L-threonine, using strains of the Enterobacteriaceae family, in addition to overexpressing the galP gene, to overexpress one or more enzymes in the well-known threonine biosynthesis pathway or enzymes from anoplerotic metabolism or enzymes for the production of reduced nicotinamide-adenine dinucleotide phosphate or enzymes from glycolysis or PTS enzymes or enzymes from sulfur metabolism. The use of endogeneous genes is generally preferred.

Thus, for example, one or more genes chosen from the group
- the thrABC operon coding for aspartate kinase, homos erinedehydrogenase, homoserine kinase and threonine synthase (US-A-4, 278, 765),
- the pyc gene from Corynebacterium glutamicum coding for pyruvate carboxylase (WO 99/18228),
- the pps gene coding for phosphoenolpyruvate synthase (Molecular and General Genetics 231(2): 332-336 (1992)),
- the ppc gene coding for phosphoenolpyruvate carboxylase (Gene 31: 279-283 (1984)),
- the pntA and pntB genes coding for transhydrogenase (European Journal of Biochemistry 158: 647-653 (1986)),
- the rhtB gene which imparts homoserine resistance (EP-A-0 994 190),
- the mqo gene coding for malate:quinone oxidoreductase (WO 02/06459),
- the rhtC gene which imparts threonine resistance (EP-A-1 013 765),
- the thrE gene from Corynebacterium glutamicum coding for threonine export protein (WO 01/92545),
- the gdhA gene coding for glutamate dehydrogenase (Nucleic Acids Research 11: 5257-5266 (1983); Gene 23: 199-209 (1983)),
- the glk gene coding for glucokinase (Journal of Bacteriology 179: 1298-1306 (1997),
- the hns gene coding for DNA binding protein HLP-II (WO 03/004671),
- the pgm gene coding for phosphoglucomutase (WO 03/004598),
- the fba gene coding for fructose biphosphate aldolase (WO 03/004664),
- the ptsH gene from the ptsHIcrr operon coding for phosphohistidine protein hexose phosphotransferase in the phosphotransferase system PTS (WO 03/004674),
- the ptsI gene from the ptsHIcrr operon coding for enzyme I in the phosphotransferase system PTS (WO 03/004674),
- the crr gene from the ptsHIcrr operon coding for the glucose-specific IIA component in the phosphotransferase systems PTS (WO 03/004674),
- the ptsG gene coding for the glucose-specific IIBC component (WO 03/004670),
- the lrp gene coding for the regulator in the leucine regulon (WO 03/004665),
- the csrA gene coding for the global regulator Csr (Journal of Bacteriology 175: 4744-4755 (1993),
- the fadR gene coding for the regulator in the fad regulon (Nucleic Acids Research 16: 7995-8009 (1988)),
- the ic1R gene coding for the regulator in the central intermediary metabolism (Journal of Bacteriology 172: 2642-2649 (1990)),
- the mopB gene coding for the 10 KDa chaperone (WO 03/004669), which is also known under the name groES,
- the ahpC gene from the ahpCF operon coding for the small sub-unit of alkyl hydroperoxide reductase (WO 03/004663),
- the ahpF gene from the ahpCF operon coding for the large sub-unit of alkyl hydroperoxide reductase (WO 03/004663),
- the cysK gene coding for cysteine synthase A (WO 03/006666),
- the cysB gene coding for the regulator in the cys regulon (WO 03/006666),
- the cysJ gene from the cysJIH operon coding for the flavoprotein in NADPH sulfite reductase (WO 03/006666),
- the cysI gene from the cysJIH operon coding for haemoprotein in NADPH sulfite reductase (WO 03/006666),
- the cysH gene from the cysJIH operon coding for adenylylsulfate reductase (WO 03/006666),
- the phoB gene from the phoBR operon coding for the positive regulator PhoB in the pho regulon (WO 03/008606),
- the phoR gene from the phoBR operon coding for the sensor protein in the pho regulon (WO 03/008606),
- the phoE gene coding for protein E in the outer cell membrane (WO 03/008608),
- the pykF gene coding for the pyruvate kinase I stimulated by fructose (WO 03/008609),
- the pfkB gene coding for 6-phosphofructokinase II (WO 03/008610),
- the malE gene coding for periplasmatic binding protein in maltose transport (WO 03/008605),
- the sodA gene coding for superoxidedismutase (WO 03/008613),
- the rseA gene from the rseABC operon coding for a membrane protein with anti-sigmaE activity (WO 03/008612),
- the rseC gene from the rseABC operon coding for a global regulator in the sigmaE factor (WO 03/008612),
- the sucA gene from the sucABCD operon coding for the decarboxylase sub-unit of 2-ketoglutarate dehydrogenase (WO 03/008614),
- the sucB gene from the sucABCD operon coding for the dihydrolipoyltranssuccinase E2 sub-unit of 2-ketoglutarate dehydrogenase (WO 03/008614),
- the sucC gene from the sucABCD operon coding for the (β-sub-unit of succinyl-CoA synthetase (WO 03/008615),
- the sucD gene from the sucABCD operon coding for the α-sub-unit in succinyl-CoA synthetase (WO 03/008615),
- the adk gene coding for adenylate kinase (Nucleic Acids Research 13(19): 7139-7151 (1985)),
- the hdeA gene coding for a periplasmatic protein with a chaperonin-like function (Journal of Bacteriology 175(23): 7747-7748 (1993)),
- the hdeB gene coding for a periplasmatic protein with a chaperonin-like function (Journal of Bacteriology 175(23): 7747-7748 (1993)),
- the icd gene coding for isocitrate dehydrogenase (Journal of Biological Chemistry 262(22): 10422-10425 (1987)),
- the mglB gene coding for periplasmatic, galactose-binding transport protein (Molecular and General Genetics 229(3): 453-459 (1991)),
- the lpd gene coding for dihydrolipoamide dehydrogenase (European Journal of Biochemistry 135(3): 519-527 (1983)),
- the aceE gene coding for the E1 component of pyruvate dehydrogenase complex (European Journal of Biochemistry 133(1): 155-162 (1983)),
- the aceF gene coding for the E2 component of pyruvate dehydrogenase complex (European Journal of Biochemistry 133(3): 481-489 (1983)),
- the pepB gene coding for aminopeptidase B (Journal of Fermentation and Bioengineering 82: 392-397 (1996))
- the aldH gene coding for aldehyde dehydrogenase (E.C. 1.2.1.3) (Gene 99(1): 15-23 (1991)),
- the bfr gene coding for the iron storage homoprotein • (bacterioferritin) (Journal of Bacteriology 171(7): 3940-3947 (1989)),
- the udp gene coding for uridine phosphorylase (Nucleic Acids Research 17(16): 6741 (1989)) and
- the rseB gene coding for the regulator of sigmaE factor activity (Molecular Microbiology 24(2): 355-371 (1997))
may be simultaneously overexpressed.

Furthermore, it may be advantageous for the production of the above-mentioned L-amino-acids in addition to overexpressing the galP gene, to attenuate, in particular to switch off or reduce the expression of, one or more of the genes chosen from the group
- the tdh gene coding for threonine dehydrogenase (Journal of Bacteriology 169: 4716-4721 (1987)),
- the mdh gene coding for malate dehydrogenase (E.C. 1.1.1.37) (Archives in Microbiology 149: 36-42 (1987)),
- the gene product of the open reading frame (ORF) yjfA (Accession Number AAC77180 at the National Center for Biotechnology Information (NCBI, Bethesda, MD, USA, WO 02/29080),
- the gene product of the open reading frame (ORF) ytfP • (Accession Number AAC77179 at the National Center for Biotechnology Information (NCBI, Bethesda, MD, USA, WO 02/29080),
- the pckA gene coding for the enzyme phosphoenolpyruvate carboxykinase (WO 02/29080),
- the poxB gene coding for pyruvate oxidase (WO 02/36797),
- the aceA gene coding for the enzyme isocitrate lyase (WO 02/081722),
- the dgsA gene coding for the DgsA regulator in the phosphotransferase system (WO 02/081721), which is also known under the name mlc gene,
- the fruR gene coding for fructose repressor (WO 02/081698), which is also known under the name cra gene,
- the rpo S gene coding for the sigma³⁸-Factor (WO 01/05939), which is also known under the name katF gene,
- the aspA gene coding for aspartate ammonium lyase
- (WO 03/008603) and
- the aceB gene coding for malate synthase A (WO 03/008603).

The expression "attenuation" in this connection describes the reduction in or switching off of intracellular activity or concentration of one or more enzymes or proteins in a microorganism, these being coded by the corresponding DNA, by, for example, using a weak promoter or a gene or allele which codes for a corresponding enzyme or protein with a lower activity or by inactivating the corresponding enzyme or protein or gene and optionally by combining these measures.

Due to the measure of attenuation, the activity or concentration of the corresponding protein is generally lowered to 0 to 75%, 0 to 50%, 0 to 25%, 0 to 10% or 0 to 5% of the activity or concentration of the wild-type protein in the starting microorganism.

Furthermore, it may be advantageous for the production of L-amino acids, in particular L-threonine, in addition to overexpressing the galP gene, to switch off undesired side reactions (Nakayama: "Breeding of Amino Acid Producing Microorganisms", in: Overproduction of Microbial Products, Krumphanzl, Sikyta, Vanek (eds.), Academic Press, London, UK, 1982).

Microorganisms produced in accordance with the invention may be cultivated in a batch process, in a fed batch process or in a repeated fed batch process. A summary of known cultivation methods is given in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium to be used must satisfy the demands of the particular strain in an appropriate manner. Descriptions of culture media for different microorganisms are given in the manual "Manual of Methods for General Bacteriology" by the American Society for Bacteriology (Washington D.C., USA, 1981).

Suitable sources of carbon which may be used are sugar and carbohydrates such as e.g. glucose, saccharose, lactose, fructose, maltose, molasses, starch and optionally cellulose, oils and fats such as e.g. soy oil, sunflower oil, ground nut oil and coconut fat, fatty acids such as e.g. palmitic acid, stearic acid and linoleic acid, alcohols such as e.g. glycerine and ethanol and organic acids such as e.g. acetic acid. These substances may be used separately or as a mixture.

Sources of nitrogen which may be used are organic nitrogen-containing compounds such as peptones, yeast extract, meat extract, malt extract, corn steep water, soy bean flour and urea or inorganic compounds such as ammonium sulfate, ammonium chloride, ammonium phosphate, ammonium carbonate and ammonium nitrate. The sources of nitrogen may be used separately or as a mixture.

Sources of phosphorus which may be used are phosphoric acid, potassium dihydrogen phosphate or dipotassium hydrogen phosphate or the corresponding sodium-containing salts. The culture medium also has to contain salts of metals such as e.g. magnesium sulfate or iron sulfate, which are needed for growth. Finally, essential growth substances such as amino acids and vitamins may also be used in addition to the substances mentioned above. Suitable precursors may also be added to the culture medium. The feedstocks mentioned above may be added to the culture in the form of a single mixture or may be fed during cultivation in an appropriate manner.

Fermentation is generally performed at a pH of 5.5 to 9.0, in particular 6.0 to 8.0. Basic compounds such as sodium hydroxide, potassium hydroxide, ammonia and ammonia water or acid compounds such as phosphoric acid or sulfuric acid are used in an appropriate manner to control the pH. Antifoam agents such as e.g. fatty acid polyglycol esters can be used to control the formation of foam. Substances which act in a selective manner, such as e.g. antibiotics, can be added to the medium to maintain stability of the plasmids. In order to maintain aerobic conditions, oxygen or oxygen-containing gases, such as e.g. air, are introduced to the culture. The temperature of the culture is normally 25°C to 45°C and preferably 30°C to 40°C. The culture is continued until a maximum of L-amino acids or L-threonine has been produced. This objective is normally achieved within 10 hours to 160 hours. , Analysis of L-amino acids can be performed by anion exchange chromatography followed by ninhydrin derivation, as is described in Spackman et al. (Analytical Chemistry 30: 1190-1206 (1958)), or it can be performed by reversed phase HPLC, as is described in Lindroth et al. (Analytical Chemistry 51: 1167-1174 (1979)).

The process according to the invention is used for the fermentative production of L-amino acids such as, for example, L-threonine, L-isoleucine, L-valine, L-methionine, L-homoserine and L-lysine, in particular L-threonine.

The present invention is explained in more detail in the following, using working examples.

The minimal (M9) and full medium (LB) for Escherichia coli are described by J.H. Miller (A short course in bacterial genetics (1992), Cold Spring Harbor Laboratory Press). Isolation of plasmid DNA from Escherichia coli and all techniques relating to restriction, ligation, Klenow and alkaline phosphatase treatments are performed as described by Sambrook et al. (Molecular Cloning - A Laboratory Manual (1989) Cold Spring Harbor Laboratory Press). Transformation of Escherichia coli is performed, unless described otherwise, as described by Chung et al. (Proceedings of the National Academy of Sciences of the United States of America 86: 2172-2175 (1989)).

The incubation temperature during the production of strains and transformants is 37°C.

### Example 1

### Construction of the expression plasmid pTrc99AgalP

The galP gene from E. coli K12 is amplified using the polymerase chain reaction (PCR) and synthetic oligonucleotides. Starting from the nucleotide sequence of the galP gene in E. coli K12 MG1655 (Accession Number AE000377, Blattner et al. (Science 277: 1453-1474 (1997)), PCR primers are synthesized. (MWG Biotech, Ebersberg, Germany). The sequences of the primer are modified so that recognition sites for restriction enzymes are produced. The recognition sequence for XbaI is chosen for the galP1 primer and the recognition site for HindIII, is chosen for the galP2 primer, these being indicated by underlining in the nucleotide sequences shown below:
galP1:
   5' - CACAATCTAGATAAACCATATTGGAGGGCATC - 3' (SEQ ID No. 1)
galP2:
   5- GGGAGGAAGCTTGGGGAGATTAATC - 3' (SEQ ID No. 2)

The chromosomal E. coli K12 MG1655 DNA used for PCR is isolated in accordance with the manufacturer's instructions, using "Qiagen Genomic-tips 100/G" (QIAGEN, Hilden, Germany). An approximately 1450 bp sized DNA fragment can be amplified with the specific primers under standard PCR conditions (Innis et al. (1990) PCR Protocols. A Guide to Methods and Applications, Academic Press) using Vent DNA polymerase (New England Biolabs GmbH, Frankfurt, Germany) (SEQ ID No. 3).

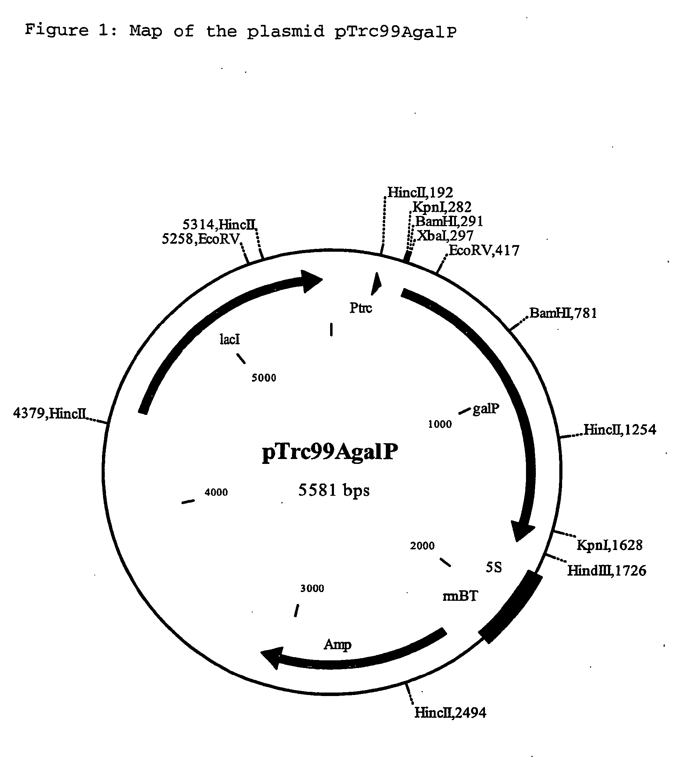

The amplified galP fragment is cleaved with the enzymes XbaI and HindIII and ligated with the vector pTrc99A (Pharmacia Biotech, Uppsala, Sweden) which has been digested with the enzymes XbaI and HindIII. The E. coli strain TOP 10 One Shot^{®} (TOPO TA Cloning Kit, Invitrogen, Groningen, Netherlands) is transformed with:the ligation mixture and plasmid-containing cells are selected on LB agar which has been treated with 50 µg/ml ampicillin. Successful cloning can be detected after plasmid DNA isolation by test cleavage with the enzymes XbaI, HindIII and EcoRV. The plasmid is called pTrc99AgalP (Figure 1).

### Example 2

Production of L-threonine with the strain MG442/pTrc99AgalP

The L-threonine producing E. coli strain MG442 is described in patent specification US-A-4,278,765 and is deposited in the Russian National Collection of Industrial Microorganisms (VKPM, Moscow, Russia) as CMIM B-1628.

The strain MG442 is transformed with the expression plasmid pTrc99AgalP described in example 1 and with the vector pTrc99A and plasmid-containing cells are selected on LB agar with 50 µg/ml ampicillin. Successful transformation can be confirmed after plasmid DNA isolation by test cleavages with the enzymes HincII, BamHI and KpnI. The strains MG442/pTrc99AgalP and MG442/pTrc99A are produced in this way. Selected individual colonies are then multiplied again on minimal medium with the following composition: 3.5 g/l Na₂HPO₄*2H₂O, 1.5 g/l KH₂PO₄, 1 g/l NH₄Cl, 0.1 g/l MgSO₄*7H₂O, 2 g/l glucose, 20 g/l agar, 50 mg/l ampicillin. The production of L-threonine is checked in batch cultures of 10 ml which are placed in 100 ml Erlenmeyer flasks. 10 ml preculture medium with the following composition is added to these: 2 g/l yeast extract, 10 g/l (NH₄)₂SO₄, 1 g/l KH₂PO₄, 0.5 g/l MgSO₄*7H₂O, 15 g/l CaCO₃, 20 g/l glucose, 50 mg/l ampicillin, inoculated and incubated for 16 hours at 37°C and 180 rpm on an ESR incubator from Kühner AG (Birsfelden, Switzerland). 250 µl of each of these precultures are then inoculated into 10 ml of production medium (25 g/l (NH₄)₂SO₄, 2 g/l KH₂PO₄ 1 g/l MgSO₄*7H₂ 0.03 g/l FeSO₄*7H₂O, 0.018 g/l MnSO₄*1H₂O, 30 g/l CaCO₃, 20 g/l glucose, 50 mg/l ampicillin) and incubated for 48 hours at 37°C. The production of L-threonine by the starting strain MG442 is checked in the same way, wherein, however, no ampicillin is added to the medium. After incubation, the optical density (OD) of the culture suspension is determined at a measurement wavelength of 660 nm using a LP2W photometer from the Dr. Lange Co. (Düsseldorf, Germany).

Finally, the concentration of L-threonine produced in sterile filtered culture supernatant liquid is determined using an amino acid analyzer from Eppendorf-BioTronik (Hamburg, Germany) by means of ion exchange chromatography and post-column reaction with ninhydrin detection.

Table 1 gives the results of the trial.

**Table 1**

| Strain | OD (660 nm) | L-threonine g/l |
|---|---|---|
| MG442 | 5.6 | 1.4 |
| MG442/pTrc99A | 3 | 1.3 |
| MG442/pTrc99AgalP | 4.1 | 1.9 |

### Brief Description of the Figure:

- Figure 1:: Map of the plasmid pTrc99AgalP containing the galP gene

Data relating to lengths are to be regarded as approximate data. The abbreviations and names used are defined as follows:
- Amp: Ampicillin resistance gene
- lacI: Gene for the repressor protein in the trc promoters
- Ptrc: trc promoter region, IPTG inducible
- galP: Coding region of the galP genes
- 5S: 5S rRNA region
- rrnBT: rRNA terminator region

The abbreviations for the restriction enzymes are defined as follows:
- BamHI: Restriction endonuclease from Bacillus amyloliquefaciens H
- EcoRV: Restriction endonuclease from Escherichia coli B946
- HincII: Restriction endonuclease from Haemophilus influence R_{c}
- HindIII: Restriction endonuclease from Haemophilus' influenzae
- KpnI: Restriction endonuclease from Klebsiella pneumoniae
- XbaI: Restriction endonuclease from Xanthomonas badrii (ATTC 11672)

## Claims

1. A process for the production of L-amino acids chosen from the group L-threonine, L-valine, L-homoserine and L-lysine, comprising:
a) fermentation of microorganisms from the genus Escherichia which produce already the desired amino acids and in which the galP gene or nucleotide sequences coding for the gene product galactose-proton symporter are overexpressed, whereby overexpression describes the increased intracellular activity or concentration of said gene product relative to the activity or concentration of said gene product in the starting microorganism, whereby the phospho transferase transport system (PTS) in said microorganisms is not inactivated compared to the corresponding wild-type PTS cell,
b) accumulation of the desired L-amino acid in the medium or in the cells of the microorganisms, and
c) isolation of the desired L-amino acid, wherein some or all (≥ 0 to 100%) of the biomass remain in the product.

2. A process according to Claim 1, wherein overexpression of the polynucleotide(s) which code(s) for the galP gene product is achieved by increasing the copy number of said gene.

3. A process according to Claim 1, wherein microorganisms are fermented in which, in addition, one or more of genes chosen from the group given below are simultaneously overexpressed:
3.1 the thrABC operon coding for aspartate kinase, homoserine dehydrogenase, homoserine kinase and threonine synthase,
3.2 the pyc gene coding for pyruvate carboxylase,
3.3 the pps gene coding for phosphoenolpyruvate synthase,
3.4 the ppc gene coding for phosphoenolpyruvate carboxylase,
3.5 the pntA and pntB genes coding for transhydrogenase,
3.6 the rhtB gene which imparts homoserine resistance,
3.7 the mqo gene coding for malate:quinone oxidoreductase,
3.8 the rhtC gene which imparts threonine resistance,
3.9 the thrE gene coding for threonine export protein,
3.10 the gdhA gene coding for glutamate dehydrogenase,
3.11 the glk gene coding for glucokinase,
3.12 the hns gene coding for DNA binding protein HLP-II,
3.13 the pgm gene coding for phosphoglucomutase,
3.14 the fba gene coding for fructose biphosphate aldolase,
3.15 the ptsH gene coding for phosphohistidine protein hexose phosphotransferase,
3.16 the ptsI gene coding for enzyme I in the phosphotransferase system,
3.17 the crr gene coding for the glucose-specific IIA component,
3.18 the ptsG gene coding for the glucose-specific IIBC component,
3.19 the lrp gene coding for the regulator in the leucine regulon,
3.20 the csrA gene coding for the global regulator Csr,
3.21 the fadR gene coding for the regulator in the fad regulon,
3.22 the iclR gene coding for the regulator in the central intermediary metabolism,
3.23 the mopB gene coding for the 10 KDa chaperone,
3.24 the ahpC gene coding for the small sub-unit of alkyl hydroperoxide reductase,
3.25 the ahpF gene coding for the large sub-unit of alkyl hydroperoxide reductase,
3.26 the cysK gene coding for cysteine synthase A,
3.27 the cysB gene coding for the regulator in the cys regulon,
3.28 the cysJ gene coding for the flavoprotein in NADPH sulfite reductase,
3.29 the cysI gene coding for haemoprotein in NADPH sulfite reductase,
3.30 the cysH gene coding for adenylylsulfate reductase
3.31 the phoB gene coding for the positive regulator PhoB in the pho regulon,
3.32 the phoR gene coding for the sensor protein in the pho regulon,
3.33 the phoE gene coding for protein E in the outer cell membrane,
3.34 the pykF gene coding for the pyruvate kinase I stimulated by fructose,
3.35 the pfkB gene coding for 6-phosphofructokinase II,
3.36 the malE gene coding for periplasmatic binding protein in maltose transport,
3.37 the sodA gene coding for superoxidedismutase,
3,38 the rseA gene coding for a membrane protein with anti-sigmaE activity,
3.39 the rseC gene coding for a global regulator in the sigmaE factor
3.40 the sucA gene coding for the decarboxylase sub-unit of 2-ketoglutarate dehydrogenase,
3.41 the sucB gene coding for the dihydrolipoyltranssuccinase E2 sub-unit of 2-ketoglutarate dehydrogenase,
3.42 the sucC gene coding for the β-sub-unit of succinyl-CoA synthetase,
3.43 the sucD gene coding for the α-sub-unit in succinyl-CoA synthetase,
3.44 the adk gene coding for adenylate kinase,
3.45 the hdeA gene coding for a periplasmatic protein with a chaperonin-like function,
3.46 the hdeB gene coding for a periplasmatic protein with a chaperonin-like function,
3.47 the icd gene coding for isocitrate dehydrogenase,
3.48 the mglB gene coding for periplasmatic, galactose-binding transport protein,
3.49 the lpd gene coding for dihydrolipoamide dehydrogenase,
3.50 the aceE gene coding for the E1 component of pyruvate dehydrogenase complex,
3.51 the aceF gene coding for the E2 component of pyruvate dehydrogenase complex,
3.52 the pepB gene coding for aminopeptidase B and
3.53 the aldH gene coding for aldehyde dehydrogenase,
3.54 the bfr gene coding for the iron storage homoprotein,
3.55 the udp gene coding for uridine phosphorylase and
3.56 the rseB gene coding for the regulator of sigmaE factor activity.

4. A process according to Claim 1, wherein microorganisms are fermented , in which, in addition, one or more of the genes chosen from the group given below are attenuated, in particular switched off or the expression of which is reduced:
4.1 the tdh gene coding for threonine dehydrogenase,
4.2 the mdh gene coding for malate dehydrogenase,
4.3 the gene product of the open reading frame (ORF) yjfA,
4.4 the gene product of the open reading frame (ORF) ytfP,
4.5 the pckA gene coding for the enzyme phosphoenolpyruvate carboxykinase;
4.6 the poxB gene coding for pyruvate oxidase,
4.7 the aceA gene coding for isocitrate lyase,
4.8 the dgsA gene coding for the DgsA regulator in the phosphotransferase system,
4.9 the fruR gene coding for fructose repressor
4.10 the rpoS gene coding for the sigma³⁸-Factor,
4.11 the aspA gene coding for aspartate ammonium lyase and
4.12 the aceB gene coding for malate synthase A gene.

## Patentansprüche

1. Verfahren zur Herstellung von L-Aminosäuren aus der Gruppe L-Threonin, L-Valin, L-Homoserin und L-Lysin, bei dem man
a) die gewünschten L-Aminosäuren bereits produzierenden Mikrooganismen der Gattung Escherichia, in denen man das ga1P-Gen oder für das Genprodukt Galaktose-Proton-Symporter kodierende Nukleotidsequenzen überexprimiert, wobei der Begriff "Überexpression" die Erhöhung der intrazellularen Aktivität oder Konzentration des genannten Genprodukts bezogen auf die Aktivität oder Konzentration des genannten Genprodukts im Ausgangs-Mikroorganismus beschreibt, wobei in den genannten Mikroorganismen das PhosphoTransferase-Transportsystem (PTS) gegenüber der entsprechenden Wildtyp-PTS-Zelle nicht inaktiviert ist, fermentiert,
b) die gewünschte L-Aminosäure im Medium oder in den Zellen der Mikroorganismen anreichert und
c) die gewünschte L-Aminosäure isoliert, wobei die Biomasse in ihrer Gesamtheit oder Anteilen (≥ 0 bis 100%) im Produkt verbleiben.

2. Verfahren nach Anspruch 1, bei dem man zur Überexpression der für das Genprodukt galP kodierende Polynukleotidsequenz(en) die Kopienzahl des genannten Gens erhöht.

3. Verfahren nach Anspruch 1, bei dem man Mikroorganismen fermentiert, in denen man zusätzlich gleichzeitig eines oder mehrere der Gene, ausgewählt aus der Gruppe:
3.1 das für die Aspartatkinase, die Homoserin-Dehydrogenase, die Homoserinkinase und die Threoninsynthase kodierende thrABC-Operon,
3.2 das für die Pyruvat-Carboxylase kodierende pyc-Gen,
3.3 das für die Phosphoenolpyruvat-Synthase kodierende pps-Gen,
3.4 das für die Phosphoenolpyruvat-Carboxylase kodierende ppc-Gen,
3.5 die für die Transhydrogenase kodierenden Gene pntA und pntB,
3.6 das Homoserinresistenz vermittelnde Gen rhtB,
3.7 das für die Malat:Chinon Oxidoreduktase kodierende mqo-Gen,
3.8 das Threoninresistenz vermittelnde Gen rhtC,
3.9 das für das Threoninexport-Protein kodierende thrE-Gen,
3.10 das für die Glutamat-Dehydrogenase kodierende gdhA-Gen,
3.11 das für die Glucokinase kodierende Gen glk,
3.12 das für das DNA-Bindeprotein HLP-II kodierende hns-Gen,
3.13 das für die Phosphoglucomutase kodierende pgm-Gen,
3.14 das für die Fructose Biphosphat Aldolase kodierende fba-Gen,
3.15 das für die Phosphohistidin-Protein-Hexose-Phosphotransferase kodierende ptsH-Gen,
3.16 das für das Enzym I des Phosphotransferase-Systems kodierende ptsI-Gen,
3.17 das für die Glucose-spezifische IIA Komponente kodierende crr-Gen,
3.18 das für die Glucose-spezifische IIBC Komponente kodierende ptsG-Gen,
3.19 das für den Regulator des Leucin-Regulons kodierende lrp-Gen,
3.20 das für den globalen Regulator Csr kodierende csrA-Gen,
3.21 das für den Regulator des fad-Regulons kodierende fadR-Gen,
3.22 das für den Regulator des zentralen Intermediärstoffwechsels kodierende ic1R-Gen,
3.23 das für das 10 KDa Chaperon kodierende mopB-Gen,
3.24 das für die kleine Untereinheit der Alkyl Hydroperoxid Reduktase kodierende ahpC-Gen,
3.25 das für die große Untereinheit der Alkyl Hydroperoxid Reduktase kodierende ahpF-Gen,
3.26 das für die Cystein-Synthase A kodierende cysK-Gen,
3.27 das für den Regulator des cys-Regulons kodierende cysB-Gen,
3.28 das für das Flavoprotein der NADPH-Sulfit-Reduktase kodierende cysJ-Gen,
3.29 das für das Hämoprotein der NADPH-Sulfit-Reduktase kodierende cysI-Gen,
3.30 das für die Adenylylsulfat-Reduktase kodierende cysH-Gen,
3.31 das für den positiven Regulator PhoB des pho Regulons kodierende phoB-Gen,
3.32 das für das Sensorprotein des pho Regulons kodierende phoR-Gen,
3.33 das für das Protein E der äußeren Zellmembran kodierende phoE-Gen,
3.34 das für die, durch Fructose stimulierte, Pyruvat Kinase I kodierende pykF-Gen,
3.35 das für die 6-Phosphofructokinase II kodierende pfkB-Gen,
3.36 das für das periplasmatische Bindeprotein des Maltose-Transports kodierende malE-Gen,
3.37 das für die Superoxiddismutase kodierende sodA-Gen,
3.38 das für ein Membranprotein mit anti-sigmaE-Aktivität kodierende rseA-Gen,
3.39 das für einen globalen Regulator des sigmaE-Faktors kodierende rseC-Gen,
3.40 das für die Decarboxylase Untereinheit der 2-Ketoglutarat Dehydrogenase kodierende sucA-Gen,
3.41 das für die Dihydrolipoyltranssuccinase E2 Untereinheit der 2-Ketoglutarat Dehydrogenase kodierende sucB-Gen,
3.42 das für die β-Untereinheit der Succinyl-CoA Synthetase kodierende sucC-Gen,
3.43 das für die α-Untereinheit der Succinyl-CoA Synthetase kodierende sucD-Gen,
3.44 das für die Adenylat-Kinase kodierende adk-Gen,
3.45 das für ein periplasmatisches Protein mit Chaperonin-artiger Funktion kodierende hdeA-Gen,
3.46 das für ein periplasmatisches Protein mit Chaperonin-artiger Funktion kodierende hdeB-Gen,
3.47 das für die Isocitrat-Dehydrogenase kodierende icd-Gen,
3.48 das für das periplasmatische, Galaktosebindende Transportprotein kodierende mg1B-Gen,
3.49 das für die Dihydrolipoamid-Dehydrogenase kodierende lpd-Gen,
3.50 das für die E1-Komponente des Pyruvat-Dehydrogenase-Komplexes kodierende aceE-Gen,
3.51 das für die E2-Komponente des Pyruvat-Dehydrogenase-Komplexes kodierende aceF-Gen,
3.52 das für die Aminopeptidase B kodierende pepB-Gen und
3.53 das für die Aldehyd-Dehydrogenase kodierende a1dH-Gen,
3.54 das für das Eisen-Speicher-Homoprotein kodierende bfr-Gen,
3.55 das für die Uridin-Phosphorylase kodierende udp-Gen und
3.56 das für den Regulator der SigmaE-Faktor-Aktivität kodierende rseB-Gen überexprimiert.

4. Verfahren nach Anspruch 1, bei dem man Mikroorganismen fermentiert, in denen man zusätzlich gleichzeitig eines oder mehrere der Gene, ausgewählt aus der Gruppe:
4.1 das für die Threonin-Dehydrogenase kodierende tdh-Gen,
4.2 das für die Malat-Dehydrogenase kodierende mdh-Gen,
4.3 das Genprodukt des offenen Leserahmens (orf) yjfA,
4.4 das Genprodukt des offenen Leserahmens (orf) ytfP,
4.5 das für die Phosphoenolpyruvat-Carboxykinase kodierende pckA-Gen,
4.6 das für die Pyruvat-Oxidase kodierende poxB-Gen,
4.7 das für die Isocitrat-Lyase kodierende aceA-Gen,
4.8 das für den DgsA-Regulator des Phosphotransferase-Systems kodierende dgsA-Gen,
4.9 das für den Fructose-Repressor kodierende fruR-Gen,
4.10 das für den Sigma38-Faktor kodierende rpoS-Gen,
4.11 das für die Aspartat Ammonium-Lyase kodierende aspA-Gen und
4.12 das für die Malatsynthase A kodierende aceB-Gen
abschwächt, insbesondere ausschaltet oder die Expression verringert.

## Revendications

1. Processus pour la production d'acides L-aminés choisis dans le groupe de la L-thréonine, la L-valine, la L-homosérine et la L-lysine, comprenant
a) une fermentation de microorganismes provenant du genre Escherichia, qui produisent déjà les acides aminés souhaités et dans lesquels le gène galP ou des séquences nucléotidiques codant pour le produit génique symporteur à protons de galactose est (sont) surexprimé(es), moyennant quoi une surexpression décrit l'activité ou la concentration intracellulaire accrue dudit produit génique par rapport à l'activité ou la concentration dudit produit génique dans le microorganisme de départ, moyennant quoi le système de transport à phosphotransférase (PTS) dans lesdits microorganismes n'est pas activé en comparaison avec la cellule à PTS de type sauvage,
b) une accumulation de l'acide L-aminé souhaité dans le milieu ou dans les cellules des microorganismes et
c) un isolement de l'acide L-aminé souhaité, moyennant quoi tout ou partie (≥ 0 jusqu'à 100%) de la biomasse reste dans le produit.

2. Processus selon la revendication 1, dans lequel une surexpression du (des) polynucléotide(s) qui code(codent) pour le produit génique de galP est accomplie en augmentant le nombre de copies dudit gène.

3. Processus selon la revendication 1, dans lequel des microorganismes sont fermentés, dans lesquels un ou plusieurs gène(s) choisi(s) dans le groupe cité ci-dessous est(sont) en plus surexprimé(s) simultanément:
3.1 L'opéron thrABC codant pour une aspartate kinase, une homosérine déshydrogénase, une homosérine kinase et une thréonine synthase,
3.2 Le gène pyc codant pour une pyruvate carboxylase,
3.3 Le gène pps codant pour une phosphoénolpyruvate synthase,
3.4 Le gène ppc codant pour une phosphoénolpyruvate carboxylase,
3.5 Les gènes pntA et pntB codant pour une transhydrogénase,
3.6 Le gène rhtB qui confère une résistance à l'homosérine,
3.7 Le gène mqo codant pour une malate:quinone oxydoréductase,
3.8 Le gène rhtC qui confère une résistance à la thréonine,
3.9 Le gène thrE codant pour une protéine d'exportation de la thréonine,
3.10 Le gène gdhA codant pour une glutamate déshydrogénase,
3.11 Le gène glk codant pour une glucokinase,
3.12 Le gène hns codant pour une protéine HLP-II de liaison à l'ADN,
3.13 Le gène pgm codant une phosphoglucomutase,
3.14 Le gène fba codant pour une fructose biphosphate aldolase,
3.15 Le gène ptsH codant pour une phosphohistidine protéine hexose phosphotransférase,
3.16 Le gène ptsI codant pour une enzyme I dans le système de phosphotransférase,
3.17 Le gène crr codant pour le composant IIA spécifique du glucose,
3.18 Le gène ptsG codant pour le composant IIBC spécifique du glucose,
3.19 Le gène lrp codant pour le régulateur dans le régulon leucine,
3.20 Le gène csrA codant pour le régulateur global Csr,
3.21 Le gène fadR codant pour le régulateur dans le régulon fad,
3.22 Le gène iclR codant pour le régulateur dans le métabolisme intermédiaire central,
3.23 Le gène mopB codant pour le chaperon de 10 KDa,
3.24 Le gène ahpC codant pour la petite sous unité de l'alkyl hydroperoxyde réductase,
3.25 Le gène ahpF codant pour la grande sous unité de l'alkyl hydroperoxyde réductase,
3.26 Le gène cysK codant pour la cystéine synthase A,
3.27 Le gène cysB codant pour le régulateur dans le régulon cys,
3.28 Le gène cysJ codant pour la flavoprotéine dans une NADPH sulfite réductase,
3.29 Le gène cysI codant pour une hémoprotéine dans une NADPH sulfite réductase,
3.30 Le gène cysH codant pour une adénylylsulfate réductase,
3.31 Le gène phoB codant pour le régulateur positif PhoB dans le régulon pho,
3.32 Le gène phoR codant pour la protéine capteur dans le régulon pho,
3.33 Le gène phoE codant pour la protéine E dans la membrane cellulaire externe,
3.34 Le gène pykF codant pour la pyruvate kinase I stimulée par le fructose,
3.35 Le gène pfkB codant pour une 6-phosphofructokinase II,
3.36 Le gène malE codant pour une protéine de liaison périplasmique dans le transport du maltose,
3.37 Le gène sodA codant pour une superoxydedismutase,
3.38 Le gène rseA codant pour une protéine membranaire ayant une activité anti-sigmaE,
3.39 Le gène rseC codant pour un régulateur global dans le facteur sigmaE,
3.40 Le gène sucA codant pour la sous unité décarboxylase d'une 2-cétoglutarate déshydrogénase,
3.41 Le gène sucB codant pour la sous unité dihydrolipoyltrànssuccinase E2 d'une 2-cétoglutarate déshydrogénase,
3.42 Le gène sucC codant pour la sous unité β d'une succinyl-CoA synthétase,
3.43 Le gène sucD codant pour la sous unité α d'une succinyl-CoA synthétase,
3.44 Le gène adk codant pour une adénylate kinase,
3.45 Le gène hdeA codant pour une protéine périplasmique ayant une fonction de type chaperonine,
3.46 Le gène hdeB codant pour une protéine périplasmique ayant une fonction de type chaperonine,
3.47 Le gène icd codant pour une isocitrate désyhdrogénase,
3.48 Le gène mglB codant pour une protéine de transport périplasmique de liaison au galactose,
3.49 Le gène lpd codant pour une dihydrolipoamide déshydrogénase,
3.50 Le gène aceE codant pour le composant E1 d'un complexe de pyruvate déshydrogénase,
3.51 Le gène aceF codant pour le composant E2 d'un complexe de pyruvate déshydrogénase,
3.52 Le gène pepB codant pour une aminopeptidase B et
3.53 le gène aldH codant pour une aldéhyde déshydrogénase,
3.54 Le gène bfr codant pour une homoprotéine de stockage du fer,
3.55 Le gène udp codant pour une uridine phosphorylase et
3.56 le gène rseB codant pour le régulateur de l'activité du facteur sigmaE.

4. Processus selon la revendication 1, dans lequel des microorganismes sont fermentés, dans lesquels un ou plusieurs des gènes choisis dans le groupe cité ci-dessous est(sont) en plus atténué(s), en particulier arrêté(s), ou dont l'expression est réduite:
4.1 Le gène tdh codant pour une thréonine déshydrogénase,
4.2 Le gène mdh codant pour une malate déshydrogénase,
4.3 Le produit génique du cadre ouvert de lecture (ORF) yjfA,
4.4 Le produit génique du cadre ouvert de lecture (ORF) ytfP,
4.5 Le gène pckA codant pour l'enzyme phosphoénolpyruvate carboxykinase,
4.6 Le gène poxB codant pour une pyruvate oxydase,
4.7 Le gène aceA codant pour une isocitrate lyase,
4.8 Le gène dgsA codant pour le régulateur DgsA dans le système de phosphotransférase,
4.9 Le gène fruR codant pour un répresseur du fructose,
4.10 Le gène rpoS codant pour le facteur sigma³⁸,
4.11 Le gène aspA codant pour une aspartate ammonium lyase et
4.12 Le gène aceB codant pour un gène de la malate synthase A.
